# FASCICULE DE BREVET EUROPEEN

(11) **EP 1 713 405 B1**
(45) Date de publication et mention de la délivrance du brevet: **29.06.2016**
(21) Numéro de dépôt: 05717627.3
(22) Date de dépôt: 11.02.2005
(51) Int. Cl.: A61B 17/16

(54) **DISPOSITIF D'EXPLORATION POUR LE SUIVI DE LA PÉNÉTRATION D'UN INSTRUMENT DANS UNE STRUCTURE ANATOMIQUE**
EXPLORATIONSVORRICHTUNG ZUR ÜBERWACHUNG DES EINDRINGENS EINES INSTRUMENTS IN EINE ANATOMISCHE STRUKTUR
EXPLORATION DEVICE FOR MONITORING THE PENETRATION OF AN INSTRUMENT INTO AN ANATOMICAL STRUCTURE

(30) Priorité: 11.02.2004 FR 0401361
(43) Date de publication de la demande: 25.10.2006
(73) Titulaire: SPINEGUARD, 94160 Saint Mandé (FR)
(72) Inventeur: BOURLION, Maurice, F-42400 Saint-Chamond (FR); PETIT, Dominique, F-62180 Verton (FR); VANACKER, Gérard, Les Issambres (FR)
(74) Mandataire: Cabinet Plasseraud
(86) Numéro de dépôt international: PCT/FR2005/000338
(87) Numéro de publication internationale: WO 2005/077282

(56) Documents cités:
- FR-A- 2 835 732
- US-B1- 6 391 005

## Description

La présente invention se rapporte au domaine de la chirurgie rachidienne.

En chirurgie du rachis, par exemple lors du forage pédiculaire, il est fréquent que le cortex osseux soit traversé, cassé ou ébréché par l'instrument de forage, pouvant alors engendrer un mauvais positionnement des vis pédiculaires. Suivant ce mauvais positionnement, les vis pédiculaires, provoquent chez le patient des douleurs, paralysies, hémorragies, etc., nécessitant une nouvelle intervention chirurgicale, voire dans certains cas causant des dommages irréparables.

On connaît de la demande de brevet FR2835732, déposée par le présent demandeur, un dispositif permettant de suivre la pénétration d'un instrument (instrument de forage ou autre) dans la vertèbre par la mesure des différences d'impédance électrique au fur et à mesure de la pénétration, de sorte que le praticien sait, à chaque instant, si l'extrémité de l'instrument sort du cortex osseux et pénètre dans une zone de tissus mous (moelle, nerfs, tissus). Dans ce cas, le praticien modifie la trajectoire de l'instrument de pénétration pour revenir dans le cortex osseux. Ce document divulgue un instrument selon le préambule de la revendication 1.

Un tel dispositif permet ainsi de détecter la formation d'une brèche dans le cortex osseux au moment du forage.

Afin de faciliter le repositionnement de l'instrument de pénétration dans le cas d'une opération de forage (ou similaire, du type taraudage, perçage, ...), mais également de permettre un positionnement correct des vis pédiculaires ou de tout autre instrument chirurgical, il s'avère nécessaire pour le praticien de connaître la position exacte des brèches formées au cours du forage.

La présente invention a donc pour objet de proposer un dispositif d'exploration permettant d'indiquer la position des brèches formées au cours d'une opération de forage (ou similaire).

À cet effet, l'invention concerne dans son acceptation la plus générale un dispositif d'exploration pour le suivi de la pénétration d'un instrument dans une structure anatomique, en particulier une structure osseuse, comportant une source de tension alimentant au moins deux électrodes et un moyen de mesure de l'impédance entre lesdites électrodes, et elle est remarquable en ce que ledit dispositif comporte un moyen de localisation angulaire constitué par au moins une électrode affleurant ponctuellement une surface périphérique dudit instrument de pénétration, la surface affleurante de ladite électrode (3) ayant une position décalée par rapport à l'axe longitudinal dudit instrument, ainsi qu'un moyen de repérage de la position de ladite au moins électrode (3).

Par affleurement ponctuel, on entend une surface de contact affleurant de manière partielle et discontinue la surface périphérique dudit instrument de pénétration. Ne constitue donc notamment pas un affleurement ponctuel, une surface de contact de forme annulaire, et par extension de forme tubulaire.

Selon que l'on souhaite effectuer des mesures latéralement ou en bout de l'instrument de pénétration ou respectivement latéralement et en bout, l'instrument de pénétration sera équipé d'une au moins électrode affleurant la surface latérale dudit instrument de pénétration et/ou d'une au moins électrodes affleurant la surface périphérique de l'extrémité distale dudit instrument de pénétration.

Avantageusement, ladite électrode affleurante est entraînée en rotation, ladite électrode affleurante étant entraînée à une vitesse de rotation telle qu'elle balaye au moins 360 degrés par tranche d'enfoncement dudit instrument de pénétration dans la structure osseuse.

Selon l'invention, ledit dispositif comporte une pluralité d'électrodes affleurantes fixes espacées angulairement et en ce que le moyen de mesure d'impédance délivre un signal correspondant à chacune desdites électrodes.

Avantageusement, lesdites électrodes consistent en des contacts ponctuels espacés longitudinalement et angulairement.

Avantageusement, lesdites électrodes sont formées de bandes longitudinales.

Selon une configuration particulière de l'invention, les électrodes sont réparties autour de l'axe longitudinal de l'instrument de pénétration.

Avantageusement, les électrodes sont disposées symétriquement par rapport à l'axe longitudinal dudit instrument de pénétration.

Avantageusement, lesdites électrodes sont constituées par des tiges conductrices de section circulaire, semi-annulaire, rectangulaire et/ou triangulaire. De même, elles peuvent être constituées par des tiges conductrices excentrées.

Selon le domaine d'intervention dans lequel est utilisé l'instrument de pénétration, ledit dispositif pourra comporter à son(ses) extrémité(s) distale(s) au moins une électrode. Avantageusement, ledit dispositif comporte deux électrodes disposées à l'extrémité distale dudit instrument de pénétration, lesdites électrodes étant constitués par des tiges conductrices de section circulaire concentrique.

Avantageusement, ledit moyen de repérage consiste en un marquage visuel porté de préférence sur la poignée dudit dispositif d'exploration. Selon un mode de réalisation particulier de l'invention, ladite poignée (6) constitue ledit moyen de repérage.

Avantageusement, ledit dispositif comporte en outre un canal central pour le passage d'un instrument additionnel.

On comprendra mieux l'invention à l'aide de la description, faite ci-après à titre purement explicatif, en référence aux figures annexées :
- la figure 1 illustre une vue schématisée d'un dispositif d'exploration;
- la figure 2 illustre une vue en coupe frontale de l'extrémité distale de l'instrument de pénétration selon une première configuration;
- la figure 3 illustre une vue en coupe longitudinale de l'instrument de pénétration selon une deuxième configuration;
- la figure 4 illustre une vue en coupe frontale de l'extrémité distale de l'instrument de pénétration selon une configuration de l'invention ;
- la figure 5 illustre une vue en perspective de l'instrument de pénétration selon une deuxième configuration de l'invention ; et
- la figure 6 illustre une vue en coupe longitudinale de l'instrument de pénétration selon une autre configuration.

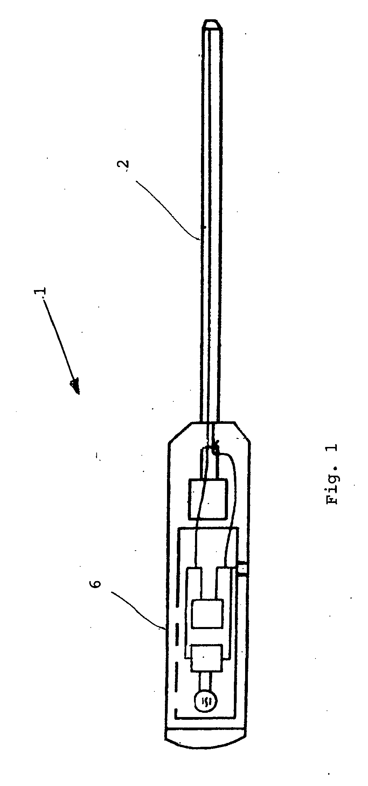

Le dispositif d'exploration (1) illustré figure 1, est un dispositif permettant le suivi de la pénétration d'un instrument (2) dans les structures osseuses d'un corps humain ou animal, lesdites structures présentant au moins deux zones d'impédance électrique différentes.

Le dispositif d'exploration (1) comporte une source de tension (non représentée) alimentant au moins deux électrodes et un moyen de mesure de l'impédance (non représenté) entre lesdites électrodes.

L'une au moins desdites électrodes est disposée sur ledit instrument de pénétration (2).

Ledit dispositif comporte en outre des moyens de signalisation produisant un signal lors de la détection, par l'impédancemètre, d'une variation d'impédance, et donc de la présence d'une brèche. Lesdits moyens de signalisation consistent à l'émission d'un signal visuel, tel qu'un témoin lumineux, d'un signal sonore, et/ou d'un signal tactile (vibreur, ...).

Selon un mode avantageux de réalisation de l'invention, ledit dispositif comporte également des moyens d'acquisition et de visualisation de la position des brèches au cours de la pénétration de l'instrument (2) dans la structure osseuse.

Selon les applications envisagées, l'instrument de pénétration (2) peut être soit fixe, soit entraîné en rotation manuellement ou par des moyens d'entraînement du type moteur (non représentés).

Ainsi, il pourra s'agir, dans la première configuration, par exemple d'une sonde, d'une pointe carrée, d'une spatule, d'une curette ou autre, et dans la seconde configuration, par exemple d'une vis, d'une mèche de forage, de taraudage, ou autre.

Dans la partie ci-après, l'instrument de pénétration (2) consiste en une sonde (2). Cependant les configurations présentées sont bien entendu applicables aux autres instruments de pénétration mentionnés ci-dessus.

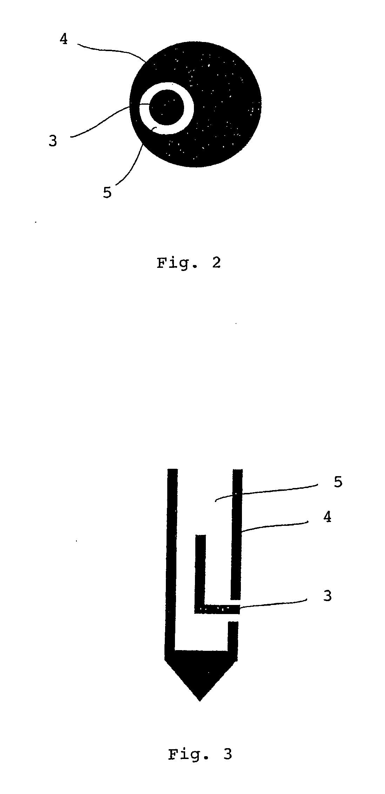

La figure 2 illustre une première configuration de la sonde (2) constituant ledit dispositif d'exploration (1).

Dans cette première configuration, l'instrument de pénétration (2) présente au niveau de son extrémité distale, deux électrodes (3, 4) de section circulaire et excentrique, l'électrode (3) étant entourée mais séparée de l'électrode (4) par une couronne d'isolant (5).

L'électrode (3) constitue, dans cet exemple qui n'est pas une réalisation de l'invention, le pôle positif dudit dispositif électronique, le pôle négatif dudit dispositif électronique étant constitué par l'électrode (4). Il est bien entendu évident qu'il ne s'agit ici que d'un exemple de réalisation, et que l'homme du métier pourra réaliser un dispositif électronique dont le pôle positif sera constitué par l'électrode (4) et le pôle négatif par l'électrode (3) sans pour autant sortir de l'invention.

Chaque électrode (3, 4) est disposée de sorte à affleurer la surface dudit instrument de pénétration (2).

Afin d'éviter toute perturbation du signal, la surface de l'électrode centrale ou interne (3) affleurant la surface dudit instrument de pénétration (2) reste relativement petite par rapport aux dimensions du trou effectué dans le cortex osseux lors de l'opération de forage (ou autre).

La position de l'électrode (3) est repérée par un marquage spécifique sur ledit dispositif d'exploration (1). Avantageusement, le marquage est effectué au moyen de la poignée (6) dudit dispositif d'exploration (1). Il pourra s'agir par exemple d'une signalisation visuelle, comme par exemple une flèche, représentée sur la poignée (6). Le marquage pourra être réalisé également au moyen directement de la poignée (6), comme par exemple une forme spécifique de ladite la poignée (6).

Ainsi, lors de la pénétration de l'instrument (2) dans la structure osseuse perforée, un signal est émis par lesdits moyens de signalisation lorsque une variation d'impédance mesurée entre les électrodes (3, 4) est détectée par l'impédancemètre, indiquant la présence d'une brèche.

Suite à cette détection, les moyens de signalisation émettent un signal d'alerte (visuel, sonore, ou tactile). Le praticien sait à ce moment que l'électrode (3) de l'instrument de pénétration est positionnée devant une brèche.

Le praticien détermine alors la direction de la brèche grâce au repère correspondant au positionnement de l'électrode (3) marqué sur la poignée (6) dudit dispositif d'exploration (1).

Afin de permettre un balayage complet de la structure osseuse, ledit instrument (2) de pénétration est animé d'un mouvement de rotation, la vitesse de rotation étant supérieure à la vitesse d'avancement de l'instrument (2) de pénétration dans la structure osseuse. En d'autres termes, la vitesse de rotation dudit instrument (2) sera telle que ledit instrument (2) de pénétration balayera au moins 360 degrés par tranche d'enfoncement.

La figure 3 illustre une seconde configuration de la sonde (2) constituant ledit dispositif d'exploration (1), laquelle permet de détecter des brèches disposées latéralement par rapport au corps dudit instrument (2) de pénétration.

Dans cette seconde configuration, l'électrode (3) est positionnée dans ledit instrument de pénétration (2) de sorte à affleurer ponctuellement la surface latérale dudit instrument (2) de pénétration.

L'électrode (4), quant à elle, est répartie sur le reste de la surface latérale restante dudit instrument (2) de pénétration, y compris son extrémité distale. Lesdites électrodes (3, 4) sont séparées l'une de l'autre par un isolant (5).

Le principe de détection et de détermination de la direction de la brèche est identique à celui exposé précédemment.

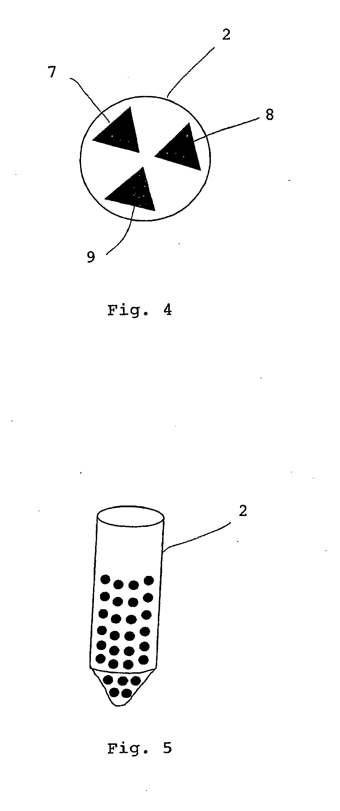

La figure 4 illustre une troisième configuration de la sonde (2) constituant ledit dispositif d'exploration (1), laquelle permet de détecter des brèches disposées à l'extrémité dudit instrument (2) de pénétration.

Dans cette troisième configuration, l'instrument de pénétration (2) présente au niveau de son extrémité distale trois électrodes (7, 8, 9) de section triangulaire sensiblement identiques. Lesdites électrodes (7, 8, 9), réparties autour de l'axe longitudinalement de l'instrument (2) de pénétration, sont espacées angulairement. Avantageusement, l'espacement angulaire est identique.

La position des électrodes (7, 8, 9) étant connue par construction, leur disposition sur l'extrémité distale donne des indications sur la position des brèches. En effet, la brèche détectée sera située entre les deux électrodes pour lesquelles un signal est émis.

Le nombre et la forme triangulaire des électrodes étant donné ici à titre d'exemple, il est entendu que ledit instrument (2) de pénétration peut présenter des électrodes en nombre supérieur et de forme autre que triangulaire. La détermination de la direction des brèches sera d'autant plus précise que le nombre d'électrodes réparties à l'extrémité dudit instrument (2) sera élevé.

La figure 5 illustre une autre configuration de la sonde (2), permettant de détecter des brèches disposées à l'extrémité dudit instrument (2) de pénétration, mais également latéralement.

Dans cette configuration, ledit instrument (2) de pénétration est constitué d'une pluralité d'électrodes disposées affleurantes à la surface latérale dudit instrument (2) de pénétration et à l'extrémité distale dudit instrument (2).

La position de chaque électrode étant connue, il est alors possible, comme pour la troisième configuration, de déterminer la position de la brèche par l'émission d'un signal par l'impédancemètre correspondant à l'électrode positionnée face à la brèche.

Dans les configurations précédemment présentées, les moyens de détermination de la position des brèches consistent en des électrodes fixes. Selon une configuration particulière de l'instrument (2) de pénétration (non représentée), la détermination des brèches pourra être également effectuée au moyen d'une ou plusieurs électrodes mobiles.

De même, dans les exemples précédents, les électrodes (3, 4) sont portées respectivement par ledit instrument de pénétration (2). Il va de soi que ledit instrument de pénétration (2) pourra être muni d'une seule électrode (3), l'autre électrode étant positionnée sur le patient, et plus particulièrement sur une surface autre que la plaie opératoire, sans pour autant sortir du champ de l'invention.

Comme cela a été précisé précédemment, les configurations présentées restent applicables aux autres instruments de pénétration mentionnés ci-dessus.

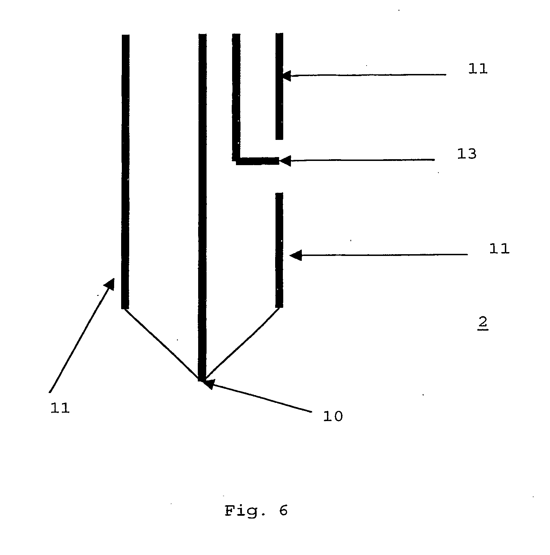

En particulier, dans le cas où l'instrument (2) de pénétration consiste en un élément de forage, ledit instrument (2) de pénétration pourra avantageusement comporter au moins une électrode (13) affleurant la surface latérale dudit instrument (2) de pénétration, ainsi que deux électrodes (10, 11) disposées concentriquement à l'extrémité distale dudit instrument (2) de pénétration (figure 6). Il sera ainsi possible, de part la configuration dudit instrument (2) de pénétration de déterminer la présence et la direction d'une brèche au moyen des électrodes (11 et 13), ainsi que de prévenir une éventuelle perforation du cortex osseux au moyen des électrodes (10 et 11). A cet effet, il devra être évité de positionner une électrode latérale consistant en une tige allant jusqu'à l'extrémité distale. Il serait en effet impossible, avec une telle configuration, de savoir si la zone détectée par les électrodes est latérale ou distale.

L'invention est décrite dans ce qui précède à titre d'exemple. Il est entendu que l'homme du métier est à même de réaliser différentes variantes de l'invention sans pour autant sortir du cadre du brevet qui est défini par les revendications.

## Revendications

1. Instrument de pénétration dans une structure anatomique, en particulier une structure osseuse, présentant une surface latérale et une extrémité distale, l'instrument de pénétration comportant un dispositif d'exploration (1) pour le suivi de la pénétration de l'instrument de pénétration (2) dans la structure anatomique, le dispositif comportant :
- au moins deux électrodes (3, 4) portées par l'instrument de pénétration,
- une source de tension alimentant lesdites électrodes (3,4) et
- un moyen de mesure de l'impédance entre lesdites électrodes (3,4),
- un moyen de localisation angulaire constitué par au moins l'une desdites électrodes (3) affleurant ponctuellement, c'est-à-dire de manière partielle et discontinue, une surface périphérique dudit instrument de pénétration (2),
- un moyen de repérage de la position de ladite au moins une électrode (3) affleurante,
dans lequel l'électrode (3) affleurante présente une surface de contact affleurant ponctuellement, c'est-à-dire de manière partielle et discontinue la surface latérale de l'instrument de pénétration (2), la surface de contact de ladite électrode (3) affleurante ayant une position décalée par rapport à un axe longitudinal dudit instrument,
l'instrument de pénétration étant **caractérisé en ce que** lesdites au moins deux électrodes (3, 4) comportent une pluralité d'électrodes (3, 4, 7, 8, 9) affleurantes comme moyen de localisation angulaire, lesdites électrodes affleurantes (3, 4, 7, 8, 9) étant fixes et espacées angulairement, et **en ce que** le moyen de mesure d'impédance délivre un signal correspondant à chacune desdites électrodes (3, 4, 7, 8, 9) affleurantes.

2. Instrument de pénétration selon la revendication 1, dans lequel au moins l'une desdites électrodes affleure ponctuellement, de manière partielle et discontinue, une surface périphérique de l'extrémité distale dudit instrument de pénétration (2).

3. Instrument de pénétration selon la revendication 1 ou 2, dans lequel lesdites électrodes (3, 4, 7, 8, 9) affleurantes consistent en des contacts ponctuels espacés longitudinalement et angulairement.

4. Instrument de pénétration selon la revendication 1 ou 2, dans lequel lesdites électrodes (3, 4, 7, 8, 9) affleurantes sont formées de bandes longitudinales.

5. Instrument de pénétration selon l'une quelconque des revendications 1 à 4, dans lequel les électrodes (3, 4, 7, 8, 9) affleurantes sont réparties autour de l'axe longitudinal de l'instrument de pénétration (2).

6. Instrument de pénétration selon l'une quelconque des revendications 1 à 5, dans lequel les électrodes (3, 4, 7, 8, 9) affleurantes sont disposées symétriquement par rapport à l'axe longitudinal dudit instrument de pénétration (2).

7. Instrument de pénétration selon l'une quelconque des revendications 1 à 6, dans lequel lesdites électrodes (3, 4, 7, 8, 9) affleurantes sont constituées par des tiges conductrices de section circulaire, semi- annulaire, rectangulaire et/ou triangulaire.

8. Instrument de pénétration selon l'une quelconque des revendications 1 à 7, dans lequel lesdites électrodes affleurantes sont constituées par des tiges conductrices excentrées.

9. Instrument de pénétration selon l'une quelconque des revendications précédentes, dans lequel au moins l'une des électrodes est disposée à l'extrémité distale dudit instrument de pénétration.

10. Instrument de pénétration selon la revendication précédente, dans lequel lesdites au moins deux électrodes comprennent deux électrodes disposées à l'extrémité distale dudit instrument de pénétration, lesdites électrodes étant constituées par des tiges conductrices de section circulaire concentrique.

11. Instrument de pénétration selon l'une quelconque des revendications précédentes, dans lequel ledit moyen de repérage consiste en un marquage visuel porté sur le dispositif d'exploration (1).

12. Instrument de pénétration selon l'une quelconque des revendications précédentes, dans lequel le dispositif d'exploration (1) comporte une poignée (6) constituant ledit moyen de repérage.

13. Instrument de pénétration selon la revendication 12 lorsqu'elle dépend de la revendication 11, dans lequel le marquage visuel est porté par la poignée (6).

14. Instrument de pénétration selon l'une quelconque des revendications précédentes, comportant un canal central pour le passage d'un instrument additionnel.

## Patentansprüche

1. Instrument zum Eindringen in eine anatomische Struktur, insbesondere eine Knochenstruktur, welches eine laterale Fläche und ein distales Ende aufweist, wobei das Instrument zum Eindringen eine Explorationsvorrichtung (1) zum Nachfolgen des Eindringens des Instruments zum Eindringen (2) in die anatomische Struktur umfasst, wobei die Vorrichtung umfasst:
- wenigstens zwei Elektroden (3, 4), welche von dem Instrument zum Eindringen getragen sind,
- eine Spannungsquelle, welche die Elektroden (3, 4) versorgt, und
- ein Mittel zum Messen der Impedanz zwischen den Elektroden (3, 4),
- ein Mittel zur Winkellokalisierung, welches durch wenigstens eine der Elektroden (3) gebildet ist, welche punktförmig, das heißt teilweise und eine Umfangsfläche des Instruments zum Eindringen (2) unterbrechend freiliegt,
- ein Mittel zum Bestimmen der Position der wenigstens einen freiliegenden Elektrode (3),
wobei die freiliegende Elektrode (3) eine punktförmig freiliegende Kontaktfläche aufweist, das heißt teilweise und die laterale Fläche des Instruments zum Eindringen (2) unterbrechend, wobei die Kontaktfläche der freiliegenden Elektrode (3) eine bezüglich einer longitudinalen Achse des Instruments verlagerte Position aufweist,
wobei das Instrument zum Eindringen **dadurch gekennzeichnet ist, dass** die wenigstens zwei Elektroden (3, 4) eine Mehrzahl von freiliegenden Elektroden (3, 4, 7, 8, 9) als Mittel zur Winkellokalisierung umfassen, wobei die freiliegenden Elektroden (3, 4, 7, 8, 9) fest und winkelbeabstandet sind, und dass das Mittel zum Messen der Impedanz ein Signal liefert, welches jeder der freiliegenden Elektroden (3, 4, 7, 8, 9) entspricht.

2. Instrument zum Eindringen nach Anspruch 1, wobei wenigstens eine der Elektroden derart teilweise punktförmig freiliegt, dass sie eine Umfangsfläche des distalen Endes der Instruments zum Eindringen (2) unterbricht.

3. Instrument zum Eindringen nach Anspruch 1 oder 2, wobei die freiliegenden Elektroden (3, 4, 7, 8, 9) aus punktförmigen Kontakten bestehen, welche longitudinal und winkelbeabstandet sind.

4. Instrument zum Eindringen nach Anspruch 1 oder 2, wobei die freiliegenden Elektroden (3, 4, 7, 8, 9) durch longitudinale Bänder gebildet sind.

5. Instrument zum Eindringen nach einem der Ansprüche 1 bis 4, wobei die freiliegenden Elektroden (3, 4, 7, 8, 9) um die longitudinale Achse des Instruments zum Eindringen (2) verteilt sind.

6. Instrument zum Eindringen nach einem der Ansprüche 1 bis 5, wobei die freiliegenden Elektroden (3, 4, 7, 8, 9) symmetrisch bezüglich der longitudinalen Achse des Instruments zum Eindringen (2) angeordnet sind.

7. Instrument zum Eindringen nach einem der Ansprüche 1 bis 6, wobei die freiliegenden Elektroden (3, 4, 7, 8, 9) durch leitfähige Stäbe mit kreisförmigem, halb-ringförmigem, rechteckigem oder/und dreieckigem Querschnitt gebildet sind.

8. Instrument zum Eindringen nach einem der Ansprüche 1 bis 7, wobei die freiliegenden Elektroden durch exzentrische leitfähige Stäbe gebildet sind.

9. Instrument zum Eindringen nach einem der vorhergehenden Ansprüche, wobei wenigstens eine der Elektroden an dem distalen Ende des Instruments zum Eindringen angeordnet ist.

10. Instrument zum Eindringen nach dem vorhergehenden Anspruch, wobei die wenigstens zwei Elektroden zwei Elektroden umfassen, welche an dem distalen Ende des Instruments zum Eindringen angeordnet sind, welche Elektroden durch leitfähige Stäbe mit konzentrischem kreisförmigem Querschnitt gebildet sind.

11. Instrument zum Eindringen nach einem der vorhergehenden Ansprüche, wobei das Mittel zum Bestimmen aus einer visuellen Markierung besteht, welche an der Explorationsvorrichtung (1) getragen ist.

12. Instrument zum Eindringen nach einem der vorhergehenden Ansprüche, wobei die Explorationsvorrichtung (1) einen Griff (6) umfasst, welcher das Mittel zum Bestimmen bildet.

13. Instrument zum Eindringen nach Anspruch 12, wenn dieser von Anspruch 11 abhängt, wobei die visuelle Markierung von dem Griff (6) getragen ist.

14. Instrument zum Eindringen nach einem der vorhergehenden Ansprüche, umfassend einen zentralen Kanal zum Durchführen eines zusätzlichen Instruments.

## Claims

1. Instrument for penetrating an anatomical structure, in particular a bony structure, having a lateral surface and a distal end, the penetration instrument comprising an exploration device (1) for monitoring penetration of the penetration instrument (2) in the anatomical structure, the device comprising:
- at least two electrodes (3, 4) disposed on the penetration instrument,
- a voltage source supplying said electrodes (3, 4) and
- a means for measuring impedance between said electrodes (3, 4),
- an angular locating means formed by at least one of said electrodes (3) being locally flush, i.e. partially and discontinuously, with a peripheral surface of said penetration instrument (2),
- a means for locating the position of said at least one electrode (3),
wherein the flushing electrode (3) has a contact surface locally flush, i.e. partially and discontinuously, with the lateral surface of the penetration instrument (2), the contact surface of said flushing electrode (3), being in a position offset from a longitudinal axis of said instrument,
the penetration instrument being **characterised in that** said at least two electrodes (3, 4) comprise a plurality of aligned electrodes (3, 4, 7, 8, 9) as an angular locating means, said aligned electrodes (3, 4, 7, 8, 9) being fixed and angularly spaced, and **in that** the impedance measuring means supplies a corresponding signal to each of said flushing electrodes (3, 4, 7, 8,9).

2. Penetration instrument as claimed in claim 1, wherein at least one of said electrodes is locally flush, partially and discontinuously, with a peripheral surface of the distal end of said penetration instrument (2).

3. Penetration instrument as claimed in claim 1 or 2, wherein said flushing electrodes (3, 4, 7, 8, 9) comprise longitudinally and angularly spaced local contacts.

4. Penetration instrument as claimed in claim 1 or 2, wherein said flushing electrodes (3, 4, 7, 8, 9) are provided in the form of longitudinal strips.

5. Penetration instrument as claimed in any one of claims 1 to 4, wherein the flushing electrodes (3, 4, 7, 8, 9) are distributed around the longitudinal axis of the penetration instrument (2).

6. Penetration instrument as claimed in any one of claims 1 to 5, wherein the flushing electrodes (3, 4, 7, 8, 9) are disposed symmetrically with respect to the longitudinal axis of said penetration instrument (2).

7. Penetration instrument as claimed in any one of claims 1 to 6, wherein said flushing electrodes (3, 4, 7, 8, 9) are provided in the form of conductor rods with a circular, semi-annular, rectangular and/or triangular cross-section.

8. Penetration instrument as claimed in any one of claims 1 to 7, wherein said flushing electrodes are provided in the form of eccentric conductor rods.

9. Penetration instrument as claimed in any one of the preceding claims, wherein at least one of the electrodes is disposed at the distal end of said penetration instrument.

10. Penetration instrument as claimed in any one of the preceding claims, wherein said at least two electrodes comprise two electrodes disposed at the distal end of said penetration instrument, said electrodes being formed by conductor rods with a concentric circular cross-section.

11. Penetration instrument as claimed in any one of the preceding claims, wherein said locating means comprises a visual marking on the exploration device (1).

12. Penetration instrument as claimed in any one of the preceding claims, wherein the exploration device (1) comprises a handle (6) constituting said locating means.

13. Penetration instrument as claimed in claim 12 and dependent on claim 11, wherein the visual marking is disposed on the handle (6).

14. Penetration instrument as claimed in any one of the preceding claims, comprising a central passage for passing through an additional instrument.
